# EUROPÄISCHE PATENTANMELDUNG

(11) **EP 1 923 066 A1**
(43) Veröffentlichungstag der Anmeldung: **21.05.2008**
(21) Anmeldenummer: 06023192.5
(22) Anmeldetag: 08.11.2006
(51) Int. Cl.: A61K 36/48, A61K 31/201, A61P 37/08, A61P 11/06, A61P 29/00, A61P 9/00, A61P 1/00, A61P 3/10, A61Q 19/00

(54) **Eine Zusammensetzung enthaltend Rotbuschtee und konjugierte Linolsäure**

(71) Anmelder: Cognis IP Management GmbH, 40589 Düsseldorf (DE)
(72) Erfinder: Bell, Doris, 40627 Düsseldorf (DE)

(57) **Zusammenfassung**

Die vorliegende Erfindung betrifft eine Zusammensetzung enthaltend Rotbuschtee und CLA sowie deren Verwendung als Arzneimittel sowie deren kosmetische Verwendung, insbesondere als Anti-Ageing-Mittel.

## Beschreibung

Die vorliegende Erfindung betrifft eine Zusammensetzung enthaltend Rotbuschtee und CLA (konjugierte Linolsäure) sowie deren Verwendung als Arzneimittel sowie deren kosmetische Verwendung, insbesondere als Anti-Ageing-Mittel.

CLA and Rotbuschtee sind bekannt. Es ist von beiden bekannt, dass sie entzündungshemmend wirken können. Vorteilhafte Wirkungen des Rotbuschtees werden z. B. auf folgenden Seiten des Internets beschrieben:
http://www.eberlein.at/coffeeandflavor/Monatsartikel/2003/rotbuschtee.htm
http://www.harmoniousbalance.com/RooibosInfo.htm
http://www.southafrica-infoweb.com/rooibos.htm#gesuridheit
http://www.africantea.com/Rooibos-Health/rooibos-health.html
http://www.online-tee.de/rooibush_druckversion.html

ES ist bekannt, dass sowohl CLA als auch Rotbuschtee anti-inflammatorische Eigenschaften haben. Diese können u. a. dazu benutzt werden, Asthma zu behandeln oder dessen Symptome zu lindern.

Überraschenderweise wurde nun gefunden, dass CLA und Rotbuschtee in Kombination einen synergistischen Effekt zeigen, die Wirkung der Kombination ist stärker als die Summe der Wirkungen beider Substanzen allein. Dies kann an der Ergänzung der verschiedenen Wirkmechanismen beider Substanzen liegen. Rotbuschtee reduziert die Freisetzung von Histaminen, CLA reduziert die Aktivierung von Eosinophilen bzw. eosinophilem Gewebe (durch die Reduktion der Freisetzung von Cytokin IL 5, einem Entzündungsmarker). CLA reduziert Atemwegs-Hyperaktivität.

Gegenstand der vorliegenden Erfindung ist deshalb eine Zusammensetzung enthaltend Rotbuschtee und CLA.

Weiterhin ist ein Gegenstand der vorliegenden Erfindung eine Zusammensetzung enthaltend Rotbuschtee und CLA zur Verwendung als Arzneimittel.

Weiterhin ist ein Gegenstand der vorliegenden Erfindung die Verwendung einer Zusammensetzung enthaltend Rotbuschtee und CLA zur Herstellung eines Arzneimittels zur Behandlung von Allergien oder von allergischen Symptomen oder von Asthma oder von Entzündungen oder von Herzerkrankungen oder von Herzschlag oder von Schlaganfällen oder von Colitis ulcerosa oder von Diabetes II.

Weiterhin ist ein Gegenstand der vorliegenden Erfindung die kosmetische Verwendung einer Zusammensetzung enthaltend Rotbuschtee und CLA. Bevorzugt ist dabei die Verwendung als Anti-Ageing-Mittel.

CLA steht als Abkürzung für konjugierte Linolsäure (Englisch: conjugated linoleic acid), also für eine Octadecadiensäure, wobei die beiden Doppelbindungen nicht durch eines oder mehrere gesättigte C-Atome getrennt sind, sondern an den C-Atomen Nr. n und n+2 beginnen (n ist eine natürliche Zahl von 2 bis 15). Bevorzugt liegen die Doppelbindungen entweder an den Positionen 9 und 11 (9,11-Octadecadiensäure) oder an den Positionen 10 und 12 (10,12-Octadecadiensäure). Dabei ist an jeder Doppelbindung cis-trans-Isomerie (E-Z-Isomerie) möglich. Die für CLA möglichen Isomere werden durch die angaben c für cis und t für trans und durch Zahlenangaben für die Position der Doppelbindungen bezeichnet, also z. B. c9,t11-CLA.

In einer bevorzugten Ausführungsform der vorliegenden Erfindung liegt CLA als c9,t11-CLA oder als t10,c12-CLA vor.

Dabei liegt die CLA in der erfindungsgemäßen Zusammensetzung entweder als freie Säure oder als Verbindung vor. Als Verbindung kommen in Betracht Salze der CLA, z. B. die Natriumsalze, Ester der CLA, z. B. mit Glycerin, wobei das Glycerin 1, 2, oder 3 CLA-Reste tragen kann und im Übrigen Acylreste anderer Fettsäuren tragen kann.

Rotbuschtee, auch Rooibostee genannt, wächst in der Kapprovinz Südafrikas. Zur Herstellung des Tees werden die Blätter des südafrikanischen Rotbuschstrauches zerkleinert und leicht fermentiert.

Der Rotbuschtee kann in der erfindungsgemäßen Zusammensetzung enthalten sein entweder in Form der Pflanzenteile (fermentiert oder unfermentiert, bevorzugt fermentiert), aus denen man durch Aufbrühen mit Wasser den eigentlichen Tee herstellen kann, oder in Form des Tees selbst (das ist also ein wässriger Extrakt der genannten Pflanzenteile) oder in Form des entwässerten Tees.

Die erfindungsgemäße Zusammensetzung kann allergische Symptome reduzieren und Asthma-Symptome lindern.

Die erfindungsgemäße Zusammensetzung kann oral verabreicht werden. Sie kann als Medikament oder Nahrungsmittelzusatz verabreicht werden (so genanntes nutritional supplement oder functional food).

Die erfindungsgemäße Zusammensetzung kann akute und chronische Entzündungen hemmen. Sie kann das Auftreten chronischer entzündlicher Krankheiten, wie z. B. Gefäßkrankheiten, Herzschlag, Schlaganfall, Colitis ulcerosa, hemmen (also präventiv wirken). Sie kann das Auftreten degenerative Krankheiten wie z. B. Diabetes II hemmen.

Die erfindungsgemäße Zusammensetzung hat viele Vorteile. Sie kann in niedrigen Dosen verabreicht werden und trotzdem wirksam sein. Sie kann leicht verabreicht bzw. genommen werden. Sie kann z. B. in Form eines Getränks vorliegen und so leicht aufgenommen werden. Sie hat keine schweren Nebenwirkungen, wie dies bei Medikamenten, z. B. Asthma-Medikamenten oder Corticosteroiden, oft der Fall ist.

Bevorzugte Darreichungsformen der erfindungsgemäßen Zusammensetzung sind: Getränk, Tablette, Tee (insbesondere Teebeutel oder "ready to use" Eistee), Kaugummi, Schokolade, Molkereiprodukte, Müsliriegel, Kindernahrung oder Babynahrung.

Die erfindungsgemäße Zusammensetzung kann auch dazu dienen, das Auftreten von durch Entzündungen verursachter Diabetes 11 (nicht Insulin-abhängige Diabetes, NIDD) zu hemmen.

Die erfindungsgemäße Zusammensetzung kann mit weiteren kräuterbasierten Produkten kombiniert werden. In Betracht kommen zum Beispiel Bockshornklee (Samen oder Blätter), der nach Ayurveda-Tradition dazu dient, Diabetes zu behandeln, oder Opuntia ficus-indica, deren Anti-Diabetes-Wirkung bekannt ist.

Die erfindungsgemäße Zusammensetzung kann als Anti-Ageing-Mittel zur kosmetischen Behandlung eingesetzt werden. Alterserscheinungen können reduziert werden. Dies kann auf die Antioxidans-Wirkung der erfindungsgemäßen Zusammensetzung zurückgeführt werden.

## Patentansprüche

1. Eine Zusammensetzung enthaltend Rotbuschtee und CLA.

2. Die Zusammensetzung nach Anspruch 1 zur Verwendung als Arzneimittel.

3. Die Verwendung der Zusammensetzung nach Anspruch 1 zur Herstellung eines Arzneimittels zur Behandlung von Allergien oder von allergischen Symptomen.

4. Die Verwendung der Zusammensetzung nach Anspruch 1 zur Herstellung eines Arzneimittels zur Behandlung von Asthma.

5. Die Verwendung der Zusammensetzung nach Anspruch 1 zur Herstellung eines Arzneimittels zur Behandlung von Entzündungen.

6. Die Verwendung der Zusammensetzung nach Anspruch 1 zur Herstellung eines Arzneimittels zur Behandlung von Herzerkrankungen oder von Herzschlag.

7. Die Verwendung der Zusammensetzung nach Anspruch 1 zur Herstellung eines Arzneimittels zur Behandlung von Schlaganfällen oder von Colitis ulcerosa.

8. Die Verwendung der Zusammensetzung nach Anspruch 1 zur Herstellung eines Arzneimittels zur Behandlung von Diabetes II.

9. Die kosmetische Verwendung der Zusammensetzung nach Anspruch 1.

10. Die kosmetische Verwendung der Zusammensetzung nach Anspruch 1 als Anti-Ageing-Mittel.
